# FASCICULE DE BREVET EUROPEEN

(11) **EP 1 487 384 B1**
(45) Date de publication et mention de la délivrance du brevet: **08.07.2009**
(21) Numéro de dépôt: 02798770.0
(22) Date de dépôt: 18.09.2002
(51) Int. Cl.: A61F 2/46

(54) **DISPOSITIF DE MISE EN PLACE D'UN BIOMATERIAU**
VORRICHTUNG ZUM EINBRINGEN VON BIOMATERIAL
DEVICE FOR DELIVERING A BIOMATERIAL

(30) Priorité: 19.09.2001 FR 0112082
(43) Date de publication de la demande: 22.12.2004
(73) Titulaire: BIO HOLDINGS INTERNATIONAL LIMITED, Tortola (VG)
(72) Inventeur: CIROTTEAU, Yves, F-75007 PARIS (FR); GIANGRANDE, Eric, F-56300 PONTIVY (FR)
(74) Mandataire: Intes, Didier Gérard André
(86) Numéro de dépôt international: PCT/FR2002/003185
(87) Numéro de publication internationale: WO 2003/024369

(56) Documents cités:
- EP-A- 0 955 022
- DE-A- 4 022 986
- US-A- 4 671 263

## Description

La présente invention a pour objet un dispositif pour la mise en place à l'intérieur d'une partie déterminée d'un os de petites sphères d'un biomatériau.

Pour la mise en place de certains implants ou de montages orthopédiques tels qu'un système vis-plaque dans une partie localisée d'un os, par exemple, dans la tête du fémur (métaphyse fémorale), il est nécessaire tout d'abord de réaliser à l'aide d'une tarière le logement qu'occupera ultérieurement l'implant ou le montage orthopédique, c'est-à-dire la vis et le canon de la plaque, dans l'exemple particulier considéré. Pour assurer le maintien de l'implant ou du montage orthopédique dans son logement, il est nécessaire d'introduire dans celui-ci un biomatériau avant d'introduire la vis ou l'implant, afin de stabiliser le montage orthopédique, voir par exemple EP 955 022 A.

Actuellement, le remplissage du logement à l'aide du biomatériau est réalisé manuellement à l'aide d'une curette. La curette est maniée de telle façon que l'on comble progressivement le défect osseux existant. Le biomatériau est poussé à l'aide d'un chasse-greffon dans différentes directions afin de mieux répartir ce matériau dans le logement. La disposition du matériau dans le logement est contrôlée grâce à un amplificateur de brillance.

On glisse ensuite la vis sur la broche qui va ensuite progresser au milieu du biomatériau risquant soit un conflit avec le biomatériau (frottement, pulvérisation du matériau, particules métalliques pouvant se détacher de la vis) soit l'entraînement de ce matériau dans la tête fémorale, ce qui n'est pas souhaitable. En outre, la durée du comblement du logement à l'aide du biomatériau par cette technique avoisine les 15 minutes.

On comprend donc que cette technique de mise en place du biomatériau dans le logement à l'aide d'une curette et d'un chasse-greffon présente de nombreux inconvénients. En particulier, elle augmente la durée de l'intervention chirurgicale, notamment en raison des contrôles à effectuer. De plus, la bonne répartition du biomatériau dans le logement est difficile à assurer.

Il existe donc un réel besoin de disposer d'un dispositif pour la mise en place du biomatériau dans une partie prédéterminée d'un os qui permette d'accélérer le comblement du logement à l'aide du biomatériau et qui permette d'améliorer la répartition du biomatériau à l'intérieur du logement.

Pour atteindre ce but, selon l'invention, le dispositif de mise en place de petites sphères d'un biomatériau dans une partie déterminé d'un os, se **caractérise en ce qu**'il comprend :
- un corps creux cylindrique de diamètre interne D1 ayant une première extrémité de remplissage et une deuxième extrémité munie de moyens de clipsage ;
- une tige guide formant obturation comprenant une partie courante cylindrique de diamètre externe D2 et présentant à proximité de sa deuxième extrémité une portion cylindrique élargie apte à coopérer avec les moyens de clipsage du corps pour solidariser de façon temporaire ladite tige dans le corps ; et
- un piston constitué par une pièce cylindrique présentant un diamètre externe D3 et un alésage axial D4, le diamètre D3 étant légèrement inférieur au diamètre interne D1 du corps et le diamètre interne D4 étant légèrement supérieur au diamètre D2 de la tige de telle manière que, après déclipsage, ladite tige puisse coulisser de façon étanche aux sphères à l'intérieur dudit piston et que le piston puisse coulisser de façon étanche aux sphères dans l'espace annulaire entre ladite tige et ledit corps.

On comprend que grâce à la possibilité de déplacement du corps du dispositif par rapport à la tige et plus précisément par rapport à sa partie cylindrique élargie formant obturateur, on peut localiser avec précision la zone dans laquelle les sphères seront mises en place dans le logement. On comprend également que grâce à l'action du piston, dont le diamètre externe est inférieur à celui du corps et donc à celui du logement, il est possible d'obtenir une bonne répartition des sphères du biomatériau contre la paroi du logement.

Selon un mode préféré de mise en oeuvre, la tige comporte un alésage axial débouchant dans sa deuxième extrémité dépourvue d'une portion élargie sur au moins une partie de sa longueur.

L'alésage axial de la tige permet de guider l'ensemble du dispositif lors de son introduction dans le logement à l'aide d'une broche préalablement mise en place.

De préférence également, ladite première extrémité de remplissage du corps creux est prolongée par une pièce ayant un évidement de forme tronconique débouchant dans l'alésage axial dudit corps. Cette portion évasée permet de faciliter le remplissage à l'aide des sphères de l'espace annulaire entre le corps et la tige à la façon d'un entonnoir.

De préférence encore, lesdits moyens de clipsage disposés à la deuxième extrémité dudit corps comprennent des fentes longitudinales ménagées à la deuxième extrémité dudit corps et des portions en relief ménagées, à la deuxième extrémité dudit corps et faisant saillie dans ledit alésage axial, et ladite portion cylindrique élargie de la tige comporte une face latérale sensiblement cylindrique dans laquelle est ménagée une gorge apte à recevoir les portions en relief dudit corps.

D'autres caractéristiques et avantages de invention apparaîtront mieux à la lecture de la description qui suit d'un mode préféré de réalisation de l'invention donné à titre d'exemple non limitatif. La description se réfère aux figures annexées sur lesquelles :
la figure 1 est une vue de dessus du corps du dispositif de mise en place du biomatériau ;
la figure 1A est une vue de côté du corps de la figure 1 ;
la figure 2 est une vue de dessus du piston ;
la figure 2A est une vue de côté du piston de la figure 2 ;
la figure 3 est une vue de dessus de la tige avec son obturateur ;
la figure 3A est une vue de côté de la tige de la figure 3 ;
la figure 4 montre la première phase d'utilisation du dispositif consistant en la mise en place des sphères de biomatériau ;
la figure 5 montre l'étape suivante consistant dans la mise en place du piston à l'intérieur du corps du dispositif ;
la figure 6 montre l'étape suivante d'utilisation dans laquelle le corps est écarté de l'extrémité de la tige pour permettre la sortie des sphères de biomatériau ; et
la figure 7 montre l'état final du dispositif lorsque le piston vient chasser les sphères dans le logement aménagé dans l'os.

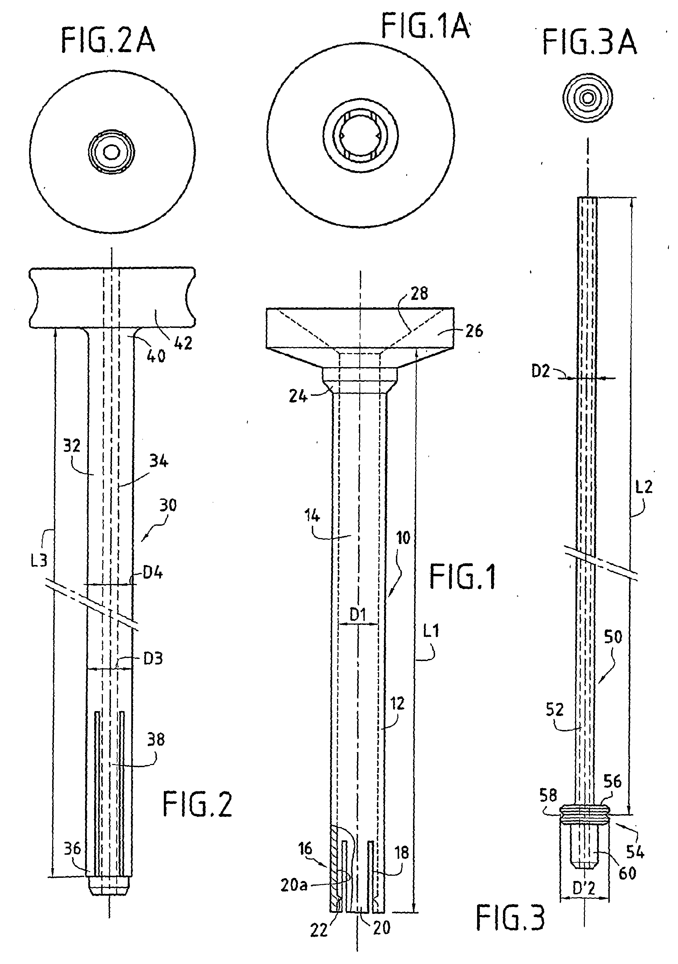

En se référant tout d'abord aux figures 1 à 3, on va décrire les différentes parties du dispositif de mise en place de sphères de biomatériau.

Sur les figures 1 et 1A, on a représenté le corps du dispositif. Ce corps 10 est constitué par une pièce tubulaire 12 présentant donc un alésage axial 14 de diamètre interne D1. Cette pièce tubulaire présente une première extrémité 16 qui est munie de fentes longitudinales telles que 18, définissant entre elles des languettes 20 élastiquement déformables. Sur leur face interne 20a, les languettes sont munies d'une portion en relief formant un ergot 22 destiné à constituer, comme on l'expliquera ultérieurement, des organes de clipsage. De préférence, la deuxième extrémité 24 du corps 10 est munie d'un prolongement évasé 26 qui définit un évidement tronconique 28 en forme d'entonnoir débouchant dans l'alésage axial 14 du corps 10.

Sur les figures 2 et 2A, on a représenté le deuxième élément du dispositif de mise en place de sphères en biomatériau constituant le piston 30. Celui-ci est également essentiellement constitué par une pièce tubulaire 32 présentant donc un alésage axial 34. Le diamètre de l'alésage axial 34 est référencé D4 alors que le diamètre externe du piston 30 est égal à D3. Le diamètre D3 est légèrement inférieur au diamètre D1 pour permettre le coulissement du piston dans le corps tout en assurant une "étanchéité" aux sphères de biomatériau. A sa première extrémité 36, de préférence le piston 30 est pourvu sur sa face externe de cannelures telles que 38 s'étendant sur une partie de sa longueur. A son autre extrémité 40, le piston 30 est muni d'une pièce de manoeuvre 42. La longueur L3 du piston 30 est supérieure à la longueur L1 du corps 10.

Sur les figures 3 et 3A, on a représenté la tige du dispositif objet de l'invention. Cette tige présente une partie courante de diamètre externe D2 et elle a une longueur L2 sensiblement supérieure aux longueurs L1 et L3 du corps et du piston. De préférence, la tige 50 présente un alésage axial 52 qui s'étend sur au moins une partie de sa longueur et de préférence sur toute sa longueur. A proximité de son extrémité 54, la tige comporte une partie de forme annulaire 56 présentant un diamètre D'2 sensiblement supérieur au diamètre D2 de la tige 50. De préférence, la portion annulaire 56 présente une gorge annulaire 58 dont la fonction sera explicitée ultérieurement. La partie terminale 60 de la tige 50 présente également un diamètre accru mais inférieur au diamètre D'2 de la partie élargie 56. Le diamètre D2 est légèrement inférieur au diamètre D4 de l'alésage axial du piston afin d'assurer une "étanchéité" aux sphères en biomatériau.

De préférence, ces différents éléments constituant le dispositif de mise en place de biomatériau sont réalisés en un acier présentant les qualités requises pour les instruments chirurgicaux.

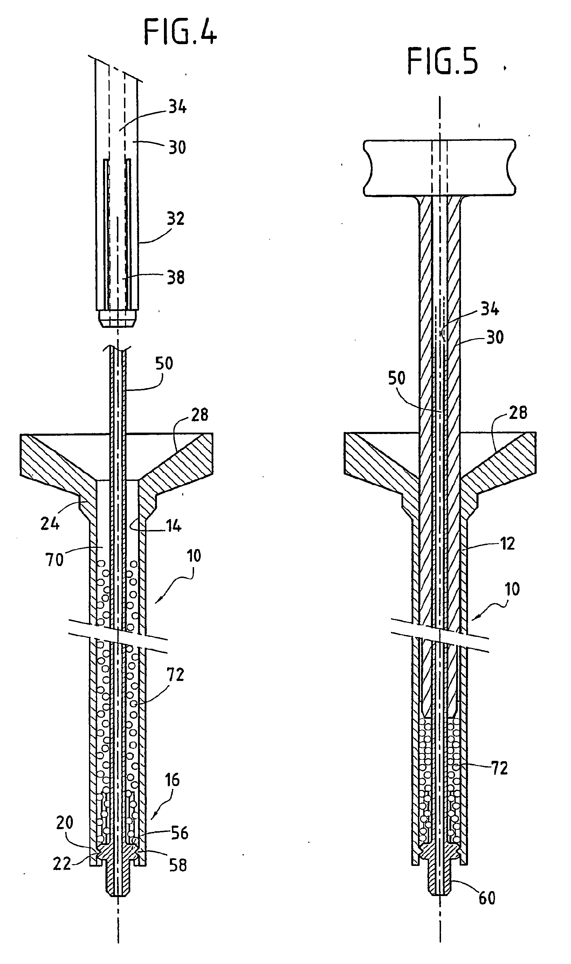

En se référant maintenant aux figures 4 à 7, on va décrire le mode d'utilisation du dispositif de mise en place de petites sphères de biomatériau dans le logement réalisé dans la partie de l'os concerné.

Sur la figure 4, on a représenté la tige 50 mise en place dans le corps 10 du dispositif. Dans cette position, la partie élargie 56 formant obturateur de la tige 50 est clipsée sur le corps 10 du dispositif par l'intermédiaire des ergots de clipsage 22 coopérant avec la gorge 58 de la portion élargie 56. Cet ensemble définit ainsi un volume annulaire 70 entre la paroi du corps 10 et la partie externe de la tige 50, ce volume étant fermé à sa partie inférieure par l'obturateur 56 de la tige 50. On introduit dans cet espace annulaire 70 à l'aide de l'entonnoir 28, la quantité nécessaire de petites sphères de biomatériau 72. Puis, comme cela est représenté sur la figure 5, on met en place le piston 30, celui-ci étant engagé par son alésage axial 34 sur la tige 50. Le piston 30 est introduit dans l'alésage axial du corps 10 de telle manière que les sphères de biomatériau 72 soient légèrement comprimées.

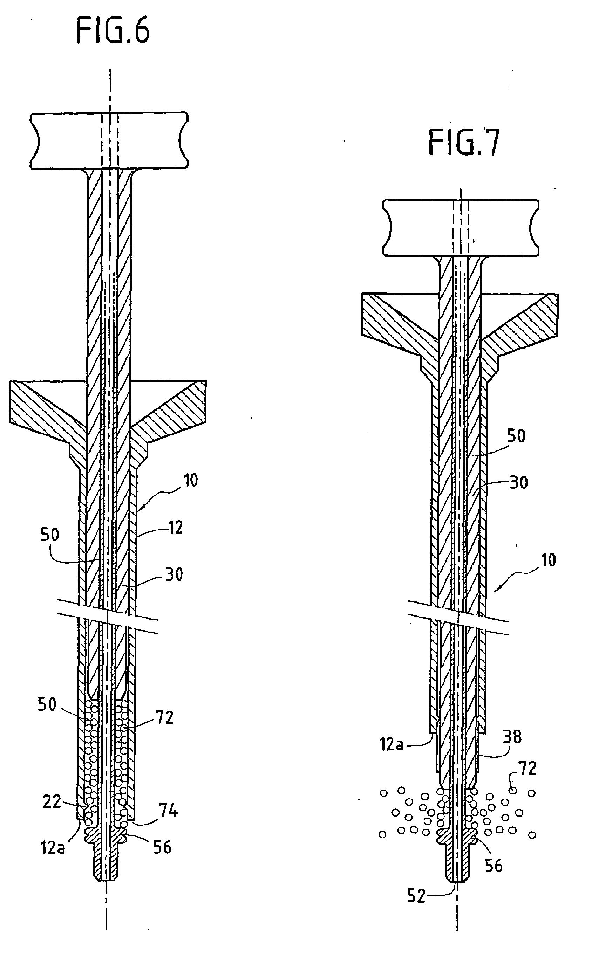

Dans l'étape suivante, représentée en partie sur la figure 6, l'ensemble du dispositif dans l'état où il est représenté sur la figure 5 est introduit dans le logement réalisé dans l'os. L'extrémité 60 de la tige constitue une limitation à l'enfoncement du dispositif dans ce logement et en assure ainsi un positionnement correct. Dans l'étape suivante, on provoque l'écartement du corps 10 par rapport à l'obturateur 56 de la tige 50, ce dernier restant immobile. Cela provoque le déclipsage de la tige par rapport au corps 10 et l'obtention d'une ouverture 74 entre l'obturateur 56 et le bord inférieur 12a du corps 10. Cette ouverture 74 permet aux sphères de biomatériau 72 de sortir du volume dans lequel elles étaient initialement confinées. En maintenant dans sa position le corps 10 et en provoquant par rapport à celui-ci l'enfoncement du piston 30, on facilite le transfert des sphères 72 à l'intérieur du logement. L'enfoncement du piston permet également de provoquer l'application des sphères 72 contre la paroi interne. Il faut ajouter qu'en provoquant la rotation du piston 30 autour de son axe, les cannelures 38 ménagées sur sa face externe permettent d'améliorer la mise en place et la répartition des sphères 72 sur la paroi du logement.

Il faut également préciser que l'alésage axial 52 prévu dans la tige 50 sert au guidage du dispositif sur la tige-broche de guidage préalablement mise en place dans le logement.

Dans un mode préféré de réalisation du dispositif, le diamètre interne D1 du corps est de l'ordre de 13 mm. Sa longueur L1 est de l'ordre de 145 mm.

Pour ce qui concerne le piston 30, son diamètre externe D3 est de l'ordre de 12,4 mm, de telle manière qu'il soit supérieur au diamètre interne D1 du corps 10, son alésage axial présente un diamètre qui est de l'ordre de 5 mm de telle manière qu'il soit légèrement supérieur au diamètre externe D2 de la tige 50 qui est de l'ordre de 4,4 mm.

Il va de soi que les différents diamètres D1, D2, D3 et D4 sont imposés par le diamètre externe du corps 10 du dispositif, le diamètre externe du corps étant lui-même fixé par le diamètre du logement réalisé dans l'os, ce logement étant le plus souvent un diamètre de 14 mm.

## Revendications

1. Dispositif de mise en place de petites sphères d'un biomatériau dans une partie déterminée d'un os, **caractérisé en ce qu'**il comprend :
. un corps creux (10) cylindrique de diamètre interne D1 ayant une première extrémité de remplissage (24) et une deuxième extrémité (16) munie de moyens de clipsage (20) ;
. une tige guide (50) formant obturateur comprenant une partie courante cylindrique de diamètre externe D2 et présentant à proximité de sa deuxième extrémité (54) une portion cylindrique élargie (56) apte à coopérer avec les moyens de clipsage (20) du corps pour solidariser de façon temporaire ladite tige dans le corps ; et
. un piston (30) constitué par une pièce cylindrique (32) présentant un diamètre externe D3 et un alésage axial de diamètre D4, le diamètre D3 étant légèrement inférieur au diamètre interne D1 du corps (10) et le diamètre interne D4 étant légèrement supérieur au diamètre D2 de la tige de telle manière que, après déclipsage, ladite tige puisse coulisser de façon étanche aux sphères à l'intérieur dudit piston et que le piston puisse coulisser de façon étanche aux sphères dans l'espace annulaire compris entre ladite tige et ledit corps.

2. Dispositif selon la revendication 1, **caractérisé en ce que** ladite tige (50) comporte un alésage axial (52) débouchant dans sa deuxième extrémité (54) pourvue d'une portion élargie (56) et s'étendant sur au moins une partie de sa longueur.

3. Dispositif selon l'une quelconque des revendications 1 et 2, **caractérisé en ce que** ladite première extrémité de remplissage (24) du corps creux (10) est prolongée par une pièce ayant un évidement de forme tronconique (28) débouchant dans l'alésage axial dudit corps.

4. Dispositif selon l'une quelconque des revendications 1 à 3, **caractérisé en ce que** lesdits moyens de clipsage disposés à la deuxième extrémité (16) dudit corps (10) comprennent des fentes longitudinales (18) ménagées à la deuxième extrémité dudit corps et des portions en relief (22) ménagées, à la deuxième extrémité dudit corps et faisant saillie dans ledit alésage axial, et **en ce que** ladite portion cylindrique élargie (56) de la tige (50) comporte une face latérale sensiblement cylindrique dans laquelle est ménagée une gorge (58) apte à recevoir les portions en relief dudit corps.

5. Dispositif selon l'une quelconque des revendications 1 à 4, **caractérisé en ce que** ledit piston (30) est muni sur sa face externe, à proximité de sa deuxième extrémité, de cannelures (38).

## Claims

1. A device for delivering small spheres of a biomaterial into a determined portion of a bone, the device being **characterized in that** it comprises:
- a cylindrical hollow body (10) of inside diameter D1 having a filling first end (24) and a second end (16) provided with snap-fastening means (20);
- a shutter-forming guide rod (50) having a main portion that is cylindrical with an outside diameter D2 and presenting, close to its second end (54), an enlarged cylindrical portion (56) suitable for co-operating with the snap-fastening means (20) of the body to secure said rod in temporary manner in said body; and
- a piston (30) constituted by a cylindrical part (32) presenting an outside diameter D3 and an axial bore of diameter D4, the diameter D3 being slightly less than the inside diameter D1 of the body (10) and the inside diameter D4 being slightly greater than the diameter D2 of the rod so that after the snap-fastening has been released, said rod can slide inside said piston and in a manner that is proof against the spheres, and the piston can slide in the annular space that exists between said rod and said body and in a manner that is proof against the spheres.

2. A device according to claim 1, **characterized in that** said rod (50) includes an axial bore (52) opening out into its second end (54) that is provided with an enlarged portion (56), the bore extending over at least a fraction of the length of the rod.

3. A device according to claim 1 or claim 2, **characterized in that** said filling first end (24) of the hollow body (10) is extended by a piece having a frustoconically-shaped recess (28) opening out into said axial bore of said body.

4. A device according to any one of claims 1 to 3, **characterized in that** said snap-fastening means disposed at the second end (16) of said body (10) comprise longitudinal slots (18) formed in the second end of said body and portions in relief (22) disposed at the second end of said body and projecting into said axial bore, and **in that** said enlarged cylindrical portion (56) of the rod (50) includes a substantially cylindrical side face in which a groove (58) is formed suitable for receiving the portions in relief on said body.

5. A device according to any one of claims 1 to 4, **characterized in that** said piston (30) is provided on its outside face, close to its second end, with fluting (38).

## Patentansprüche

1. Vorrichtung zum Einsetzen von aus einem Biomaterial bestehenden Kügelchen in einen bestimmten Teil eines Knochens, **dadurch gekennzeichnet, daß** sie folgendes umfaßt:
- einen zylindrischen Hohlkörper (10) mit einem Innendurchmesser D1, welcher ein erstes Einfüllende (24) sowie ein mit Mitteln zum Festclipsen (20) versehenes zweites Ende (16) aufweist;
- einen eine Verschlußvorrichtung bildenden Führungsschaft (50), der einen durchgehenden zylindrischen Teil mit einem Außendurchmesser D2 umfaßt und in der Nähe seines zweiten Endes (54) einen erweiterten zylindrischen Abschnitt (56) aufweist, der geeignet ist, mit den Mitteln zum Festclipsen (20) des Körpers zusammenzuwirken, um den Schaft in dem Körper vorübergehend festzulegen; sowie
- einen Kolben (30), der von einem zylindrischen Teil (32) gebildet ist, das einen Außendurchmesser D3 sowie eine Axialbohrung mit einem Durchmesser D4 aufweist, wobei der Durchmesser D3 geringfügig kleiner ist als der Innendurchmesser D1 des Körpers (10) und der Innendurchmesser D4 geringfügig größer ist als der Durchmesser D2 des Schaftes, so daß sich der Schaft nach Lösen des Clipverschlusses kügelchendicht innerhalb des Kolbens verschieben läßt und daß sich der Kolben kügelchendicht in dem ringförmigen Raum zwischen dem Schaft und dem Körper verschieben läßt.

2. Vorrichtung nach Anspruch 1, **dadurch gekennzeichnet, daß** der Schaft (50) eine Axialbohrung (52) aufweist, die in sein mit einem erweiterten Abschnitt (56) versehenes zweites Ende (54) mündet und sich über wenigstens einen Teil seiner Länge erstreckt.

3. Vorrichtung nach einem der Ansprüche 1 und 2, **dadurch gekennzeichnet, daß** das erste Einfüllende (24) des Hohlkörpers (10) durch ein Teil verlängert ist, das eine in die Axialbohrung des Körpers mündende kegelstumpfförmige Ausnehmung (28) aufweist.

4. Vorrichtung nach einem der Ansprüche 1 bis 3, **dadurch gekennzeichnet, daß** die an dem zweiten Ende (16) des Körpers (10) angeordneten Mittel zum Festclipsen Längsschlitze (18) umfassen, die an dem zweiten Ende des Körpers ausgebildet sind, sowie erhabene Abschnitte (22), die an dem zweiten Ende des Körpers ausgebildet sind und in die Axialbohrung vorspringen, und daß der erweiterte zylindrische Abschnitt (56) des Schaftes (50) eine im wesentlichen zylindrische Mantelfläche aufweist, in der eine Nut (58) ausgebildet ist, welche geeignet ist, die erhabenen Abschnitte des Körpers aufzunehmen.

5. Vorrichtung nach einem der Ansprüche 1 bis 4, **dadurch gekennzeichnet, daß** der Kolben (30) an seiner Außenseite, in der Nähe seines zweiten Endes, mit Rillen (38) versehen ist.
